# EUROPÄISCHE PATENTSCHRIFT

(11) **EP 2 030 667 B1**
(45) Veröffentlichungstag und Bekanntmachung des Hinweises auf die Patenterteilung: **16.03.2011**
(21) Anmeldenummer: 07017233.3
(22) Anmeldetag: 03.09.2007
(51) Int. Cl.: B01D 53/14, C07D 319/12, C08G 63/08

(54) **Verfahren zur Reinigung von Prozessdämpfen bei der Polylactidherstellung**
Process of cleaning process vapours in polylactide production
Procédé de lavage de vapeurs de processus lors de la fabrication de polylactide

(43) Veröffentlichungstag der Anmeldung: 04.03.2009
(73) Patentinhaber: Uhde Inventa-Fischer GmbH, 13509 Berlin (DE)
(72) Erfinder: Hagen, Rainer Dr., D-13465 Berlin (DE); Mühlbauer, Udo, D-10119 Berlin (DE); Techlin, Willi, D-12347 Berlin (DE)
(74) Vertreter: Pfenning, Meinig & Partner GbR

(56) Entgegenhaltungen:
- DE-A1- 10 257 577
- US-A- 4 835 293
- US-A- 5 266 706
- US-A- 5 856 523
- US-A1- 2001 043 898

## Beschreibung

Vorliegende Erfindung betrifft ein Verfahren zur Aufarbeitung von bei der Polylactidherstellung anfallenden Prozessdämpfen; ebenso werden Verwendungsmöglichkeiten des Verfahrens genannt.

Bei den Prozess-Stufen der Polylactidherstellung handelt es sich z. B. um die direkte Polykondensation der Milchsäure, die thermische Depolymerisation von Polylactid zu Dilactid, die Reinigung des Dilactids mittels Destillation, Rektifikation oder Kristallisation, die Polymerisation und die Entmonomerisierung. Die Dämpfe aus diesen Prozess-Stufen fallen unter verminderten Drücken bzw. Vakuum an, das zwischen 5 mbar und 200 mbar liegen kann. Sie enthalten je nach Prozess-Stufe Wasser, Milchsäure, Dilactid und Lactoylmilchsäure in unterschiedlichen Zusammensetzungen. Diese Komponenten müssen zum Schutz der Vakuumpumpen, aber auch aus prozessökonomischen Gründen soweit wie möglich kondensiert und in den Prozess zurückgeführt werden.

Die Kondensation von dilactidhaltigen Dämpfen an gekühlten Oberflächen von Kondensatoren bereitet Schwierigkeiten. Es entsteht ein Aerosol, das mit üblichen Mitteln wie Tropfen- oder Nebelabscheidern nicht niedergeschlagen werden kann, sondern den Kondensator mit dem nicht kondensierbaren Restgas verlässt und so in die Vakuumpumpen gelangt, die dieses Restgas abziehen und verdichten.

Dieses Problem verstärkt sich in dem Maße, wie die Dämpfe aus den Prozess-Stufen der Polylactidherstellung Inertgase wie Luft oder Stickstoff enthalten. Das Dilactid-Aerosol führt in Vakuumpumpen in kurzer Zeit durch erhöhten Verschleiß von metallischen Oberflächen wie Drehkolben, Drehschiebern, Sperrschiebern und deren Gehäuse zur mechanischen Zerstörung. Ein weiteres Problem ist die Umwandlung des Dilactids durch den im Restgas stets noch enthaltenen Wasserdampf in Lactoylmilchsäure, die zusammen mit den ebenfalls noch mitgeführten Milchsäureresten diese metallischen Oberflächen durch Korrosion angreifen und auf Dauer zerstören.

Die indirekte Kondensation an gekühlen Oberflächen wird in der Regel bevorzugt, da sie im Gegensatz zur direkten Kondensation mit kalten Flüssigkeiten keine zusätzlichen und unter Umständen fremden Stoffe in den Prozess einbringt und die Menge an Kondensat nicht vergrößert.

Die US 5,266,706 beschreibt einen Prozess zur Gewinnung eines cyclischen Esters wie Lactid aus einem Gasstrom, der das Lactid und hydroxylgruppenhaltige Verunreinigungen wie Wasser und Hydroxycarbonsäuren enthält, durch Waschen des Gasstroms mit einem mit Wasser nicht mischbaren Lösungsmittel wie unpolare Kohlenwasserstoffe, cycloaliphatische Kohlenwasserstoffe oder halogenierte Kohlenwasserstoffe. Dabei wird die Temperatur bei der Wäsche so eingestellt, dass der cyclische Ester und die Hydroxycarbonsäure aus dem Gasstrom entfernt wird, während Wasser im Gasstrom verbleibt und mit diesem abgeführt wird. Das rohe Gemisch aus cyclischem Ester und Säure wird vom Lösungsmittel abgetrennt und gereinigt, indem die Säure daraus extrahiert wird. Nachteilig bei dem genannten Prozess ist allerdings, dass das derart aufgereinigte Lactid nach der Aufarbeitung prozessfremder Lösungsmittel, d. h. Lösungsmittel, die nicht den im ursprünglichen Lactidstrom enthaltenen Edukten entsprechen, enthält, die im Anschluss an das Verfahren durch aufwändige Schritte wieder entfernt werden müssen. Dies ist mit hohem Aufwand und Kosten verbunden.

Aus der US 2001/0043898 A1, DE 102 57 577 A1, US-A-5,266,706 sowie der US-A-4,835,293 sind ebenfalls Vorrichtungen zur Reinigung von Prozessgasen bekannt.

Ausgehend von den Nachteilen des Standes der Technik ist es Aufgabe der vorliegenden Erfindung, ein Verfahren anzugeben, bei dem Prozessdämpfe aus verschiedenen Stufen der Polylactidherstellung kondensiert und gewaschen werden können, so dass Vakuumpumpen, die das in den einzelnen Prozess-Stufen der Polylacidherstellung benötigte Vakuum erzeugen, vor Begleitstoffen geschützt werden, die diese Pumpen chemisch (Korrosion) oder mechanisch (Abrasion) angreifen und zerstören. Dabei soll die Kondensations- und Waschflüssigkeit keine prozessfremden Stoffe in das Kondensat einschleppen, die vor einer Rückführung in den Polylactidprozess wieder abzutrennen wären.

Diese Aufgabe wird mit den Merkmalen des Patentanspruchs 1 gelöst. Dabei stellen die jeweils abhängigen Ansprüche vorteilhafte Weiterbildungen dar.

Erfindungsgemäß wird somit ein Verfahren zur Kondensation und/oder Wäsche eines dampfförmigen bioabbaubaren, intermolekularen cyclischen Diesters einer alpha-Hydroxycarbonsäure der Formel II 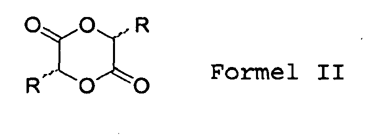 wobei R ausgewählt ist aus Wasserstoff oder linearen oder verzweigten aliphatischen Resten mit 1 bis 6 Kohlenstoffatomen,
aus einem Dampfgemisch, enthaltend den Diester der Formel II, die zum Diester der Formel II korrespondierende alpha-Hydroxycarbonsäure der Formel I und Wasser bereitgestellt, wobei ein zumindest einmaliges Inkontaktbringen eines Stromes einer Kondensations- und Waschflüssigkeit, enthaltend eine wässrige Lösung der zum Diester der Formel II korrespondierenden alpha-Hydroxycarbonsäure der Formel I 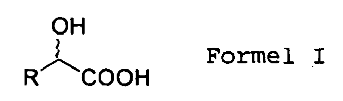 mit dem Dampfgemisch erfolgt, so dass der im Dampfgemisch enthaltene Diester der Formel II in der Kondensations- und Waschflüssigkeit gelöst wird. Das Inkontaktbringen des Dampfgemisches mit der Flüssigkeit kann dabei auf beliebige Art und Weise vollzogen werden. So ist es beispielsweise möglich, dass das Dampfgemisch in die Kondensations- und Waschflüssigkeit eingebracht wird, beispielsweise durch Einblasen oder Durchleiten, alternativ hierzu ist es aber auch möglich, wenn die Kondensations- und Waschflüssigkeit durch Berieseln, Besprühen oder Besprenkeln des Dampfgemisches kontaktiert wird.

Bevorzugt ist dabei, wenn die Kondensations- und Waschflüssigkeit in einem Kreislauf geführt wird.

Günstige Temperaturbereiche der Kondensations- und Waschflüssigkeit vor Inkontaktbringen mit dem Dampfgemisch liegen dabei zwischen 10 °C und 80 °C, bevorzugt zwischen 15 °C und 60 °C.

Um zu vermeiden, dass die Löslichkeitsgrenze des Diesters der Formel II in der Kondensations- und Waschflüssigkeit nicht überschritten wird, wird der Kondensations- und Waschflüssigkeit portionsweise oder kontinuierlich ein Gemisch aus Wasser und Hydroxycarbonsäure der Formel I zugesetzt. Dabei muss die Menge der zuzusetzenden wässrigen Lösung der Hydroxycarbonsäure der Formel I so bemessen sein, dass es nicht zur Auskristallisation des Diesters der Formel II kommt. Ebenso sollte die zugesetzte Menge derart bemessen sein, dass die Viskosität der Kondensations- und Waschflüssigkeit annähernd konstant bleibt. Die Menge bzw. Rate der zuzusetzenden Lösung hängt somit von verschiedenen Parametern, beispielsweise der Temperatur der Kondensations- und Waschflüssigkeit sowie der Menge des Diesters der Formel II in dem Dampfgemisch ab, so dass die Menge bzw. Rate der zuzusetzenden Lösung zur Herabsetzung der Konzentration des Diesters der Formel II in der Wasch- und Kondensationsflüssigkeit vom Fachmann im jeweiligen Fall mittels einfacher Experimente ermittelt kann.

Vorzugsweise wird die Konzentration des Diesters der Formel II in der Kondensations- und Waschflüssigkeit stets unter 5 Gew.-% gehalten.

Weiter ist es günstig, wenn nach Erreichen einer Konzentration von höchstens 5 Gew.-%, bevorzugt höchstens 3 Gew.-% des Diesters der Formel II in der Kondensations- und Waschflüssigkeit eine zumindest teilweise Entnahme der Kondensations- und Waschflüssigkeit erfolgt. Die Entnahme kann dabei ebenso portionsweise oder kontinuierlich erfolgen.

Eine weitere bevorzugte Ausführungsform sieht vor, dass die Kontaktierung der Waschflüssigkeit mit dem Dampfgemisch bei verminderten Drücken, insbesondere zwischen 5 mbar und 900 mbar, bevorzugt zwischen 10 mbar und 200 mbar erfolgt.

Weiter ist es vorteilhaft, wenn der Diester der Formel II Dilactid und die alpha-Hydroxycarbonsäure der Formel I Milchsäure ist. Die Erfindung ist anwendbar auf die beiden enantiomeren Formen *L,L*-Dilactid und *D,D*-Dilactid sowie *L*-Milchsäure und *D*-Milchsäure. Weiter ist sie anwendbar, wenn der Diester *D,L*-Dilactid oder Mesolactid ist.

Weiterhin ist es möglich, dass zumindest ein Teil des Diesters der Formel II aus einer vorgeschalteten Reinigungsvorrichtung stammt.

Im Folgenden wird unter Dilactid *L,L*-Dilactid, *D,D-*Dilactid, Mesolactid sowie deren Mischungen verstanden.

Ebenso kann das Dampfgemisch aus verschiedenen Prozess-Stufen bei der Polymerisation von Lactid herrühren, nämlich aus einer Prozessstufe der Herstellung von Polylactid, der Polykondensation von Milchsäure, der thermischen Depolymerisation von Oligomeren der Milchsäure mit einer mittleren Molmasse zwischen 500 g/mol und 5000 g/mol, der Rektifikation von Dilactid, der ringöffnenden Polymerisation eines dilactidhaltigen Reaktionsgemisches, der Vakuum-Entmonomerisierung von Polylacitd oder dessen Copolymeren und/oder aus mehreren der zuvor genannten Prozess-Stufen gleichzeitig.

Insbesondere ist das im Voranstehenden beschriebene Verfahren mit einer nachfolgend beschriebenen Vorrichtung durchführbar.

Zur Durchführung des Verfahrens wird eine Kondensations- und Waschvorrichtung bereitgestellt, umfassend
a) einen Sumpfbehälter, enthaltend eine Kondensations- und Waschflüssigkeit, wobei der Sumpfbehälter mindestens einen Zulauf und mindestens einen Ablauf aufweist,
b) darauf formschlüssig angebracht mindestens eine Kolonne, die mindestens eine den Querschnitt der Kolonne zumindest teilweise, bevorzugt ganz ausfüllende Stoffaustauschpackung aufweist,
c) mindestens eine Zuführung für Prozessdampf, die unterhalb der Stoffaustauschpackung der Kolonne angeordnet ist, sowie
d) mindestens eine, oberhalb der Stoffaustauschpackung der Kolonne angeordnete Abführung für Prozessdampf,
   wobei der Ablauf des Sumpfbehälters mit der Kolonne zur Gewährleistung einer Zirkulation der Kondensations- und Waschflüssigkeit über eine Rohrleitung verbunden ist und die Eintrittsrohrleitung der Kolonne oberhalb der Stoffaustauschpackung angeordnet ist.
   Bevorzugt ist es dabei, wenn die Kondensations- und Waschflüssigkeit eine wässrige Lösung einer alpha-Hydroxycarbonsäure der Formel I, 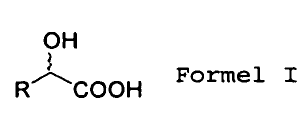 wobei R ausgewählt ist aus Wasserstoff oder linearen oder verzweigten aliphatischen Resten mit 1 bis 6 Kohlenstoffatomen, bevorzugt Milchsäure enthält. Die Konzentration der alpha-Hydroxycarbonsäure (total acidity) liegt dabei insbesondere zwischen 50 und 100 Gew.-%, bevorzugt zwischen 70 und 95 Gew.-%.
   Zusätzlich kann in der Kondensations- und Waschflüssigkeit noch ein bioabbaubarer, intermolekularer cyclischer Diester einer alpha-Hydroxycarbonsäure der Formel II, 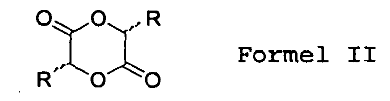 der insbesondere Dilactid ist, enthalten sein. Vorzugsweise beträgt die Konzentration des Diesters der Formel II in der Kondensations- und Waschflüssigkeit zwischen 0 und 6 Gew.-%, bevorzugt zwischen 1 und 4 Gew.-%.

Die in der Kondensations- und Waschvorrichtung enthaltene Stoffaustauschpackung umfasst dabei prinzipiell alle aus dem Stand der Technik bekannten Packungsmöglichkeiten für Kolonnen, insbesondere ist die Stoffaustauschpackung jedoch ausgewählt aus der Gruppe bestehend aus Ringen, wie z.B. Raschig- und/oder Pall-Ringen, Satteln, wie z.B. Berl-Sattel, Kugeln, Hacketten, NOR-PAC, BIO-NET, Hel-X, Top-Packs, Mellapak, Montz-Pak, Ralu-Pak, Raschig-Super-Pak und/oder Packungen aus Gewebe. Die Oberfläche der verwendeten Stoffaustauschpackungen beträgt dabei zwischen 20 m²/m³ und 500 m²/m³_{.}

In einer weiteren bevorzugten Ausführungsform weist die mindestens eine Kolonne mindestens einen Flüssigkeitsverteiler zur Verteilung der über die Rohrleitung zugeführten Kondensations- und Waschflüssigkeit auf, der oberhalb des mindestens einen Stoffaustauschpaketes angeordnet ist. Bevorzugt ist der Flüssigkeitsverteiler eine Berieselungs- oder eine Sprühvorrichtung, ein Sprühkondensator oder ein Sprinkler.

In einer weiteren Ausführungsform weisen die mindestens eine Kolonne und/oder der Sumpfbehälter Mittel zur Temperierung der Kondensations- und Waschflüssigkeit auf. Zusätzlich oder als Alternative hierzu kann es ebenso bevorzugt vorgesehen sein, dass die Rohrleitung für die Kondensationsflüssigkeit einen Wärmetauscher aufweist.

Zur Entnahme der mit dem cyclischen Diester der Formel II angereicherten Kondensations- und Waschflüssigkeit ist es bevorzugt, wenn im Sumpfbehälter eine Entnahmemöglichkeit der Kondensations- und Waschflüssigkeit vorhanden ist. Die Entnahme kann dabei portionsweise oder kontinuierlich erfolgen.

Weiterhin kann eine Polymerisationsvorrichtung zur Polymerisation des Diesters der Formel II bereitgestellt werden, die eine vorstehend beschriebene Kondensationsvorrichtung umfasst.

Vorteilhaft ist dabei, wenn der Kondensationsvorrichtung beispielsweise mindestens eine Reinigungsvorrichtung für Dilactid, die unter Vakuum betrieben wird, vorgeschaltet ist. Ebenso ist es möglich, dass der Kondensationsvorrichtung mindestens ein De-Polymerisationsreaktor vorgeschaltet ist, der unter Vakuum betrieben wird.

Verwendungsmöglichkeiten für das Verfahren ergeben sich bei der Herstellung von bioabbaubaren, intermolekularen cyclischen Diestern einer alpha-Hydroxycarbonsäure der Formel II, bevorzugt Dilactid, sowohl *L,L*-Dilactid als auch *D,D*-Dilactid und *D,L*-Dilactid (Mesolactid), sowie bei der Herstellung von Polymeren von cyclischen Diestern einer alpha-Hydroxycarbonsäure der Formel II, bevorzugt Polylactid (PLA), sowohl *L*-Polylactid (PLLA) als auch D-Polylactid (DDLA) und *D-L*-Polylactid (Polymesolactid).

Das erfindungsgemäße Verfahren ist daraufhin ausgerichtet, nicht den cyclischen Ester - das Lactid - zu gewinnen, sondern den Dampfstrom von allen kondensierbaren und abrasiven oder korrosiven Begleitstoffen zu reinigen, bevor er in eine Vakuumpumpe oder eine Serie von hintereinander geschalteten Vakuumpumpen eintritt. Die Temperatur bei der Wäsche wird so niedrig gewählt, dass einerseits ein möglichst großer Anteil der im Dampfstrom enthaltenen Komponenten auskondensiert, Wasser eingeschlossen. Andererseits wird sie so hoch gewählt, dass die Viskosität der Waschflüssigkeit nicht zu hoch wird, so dass noch eine gute Verteilung über eine Füllkörperschicht oder eine Stoffaustauschpackung möglich ist.

Das erfindungsgemäße Verfahren arbeitet nicht mit prozessfremden Lösungsmitteln, sondern im Wesentlichen mit der auskondensierten Flüssigkeit selbst, die im Kreislauf geführt wird. Dabei wird die Temperatur der Waschflüssigkeit durch im Kreislauf angeordnete Kühler eingestellt und konstant gehalten.

Überraschend wurde nun gefunden, dass die direkte Kondensation und Wäsche von dilactidhaltigen Dampfströmen aus Prozess-Stufen der Polylactidherstellung in Füllkörperschüttungen oder Stoffaustauschpackungen, die mit einer gekühlten Flüssigkeit berieselt werden, zu keiner Bildung von Aerosolen während der Kondensation führt. Als Kühlflüssigkeit hat sich ein Gemisch aus Wasser, Milchsäure, linearen Oligomeren der Milchsäure und Dilactid als geeignet erwiesen, das in den Prozess der Polylactidherstellung und dort in eine geeignete Prozess-Stufe zurückgeführt und somit wiedergewonnen werden kann. Für den Erfolg der aerosolfreien Kondensation ist die Konzentration der genannten Komponenten im flüssigen Gemisch nicht entscheidend. Im Prinzip eignet sich auch eine Mischung aus Wasser und Milchsäure dazu. Es ist jedoch zweckmäßig, sich die Konzentrationen einstellen zu lassen, die bei stationärem Betrieb des erfindungsgemäßen Kondensators und Wäschers unter vorgegebenem Vakuum und der Temperatur der Kondensations- und Waschflüssigkeit entstehen. Zum stationären Betrieb gehört einerseits die Abgabe einer der Kondensatmenge entsprechenden Menge Flüssigkeit aus dem Kreislauf. Andererseits würden dilactidhaltige Dampfströme, die im erfindungsgemäßen Prozess kondensiert werden, zu einer Anreicherung des Dilactids im Kreislauf der Kondensations- und Waschflüssigkeit führen. Diese Anreicherung führt zum Überschreiten der Löslichkeitsgrenze von Dilactid und somit zur Feststoffausscheidung in der Kreislaufflüssigkeit. Diese Feststoffausscheidung bewirkt Verstopfungen im Kreislauf uns insbesondere in der Füllkörperschüttung oder in der Stoffaustauschpackung. Außerdem reagiert das Dilactid mit dem in der Flüssigkeit enthaltenen Wasser durch Ringöffnung zur Lactoylmilchsäure. Dadurch steigt die Viskosität der Flüssigkeit und die Verteilung über die Schüttung bzw. Packung wird erschwert und die Kondensations- und Waschwirkung nimmt ab. Es ist deshalb vorteilhaft, der im Kreislauf geführten Kondensations- und Waschflüssigkeit kontinuierlich oder portionsweise ein Gemisch aus Wasser und Milchsäure zuzuspeisen, dessen Zusammensetzung und Mengenstrom so gewählt wird, dass die Löslichkeitsgrenze des Dilactids im Kreislauf nicht erreicht wird und die Viskosität des flüssigen Gemischs nicht ansteigt. Ein diesem Mengenstrom entsprechender Teilstrom der Kreislaufflüssigkeit wird zusätzlich zum Mengenstrom des Kondensats, vorzugsweise zusammen mit diesem, aus dem Kreislauf ausgeschleust und in den Polylactid-Prozess an geeigneter Stelle zurückgeführt.

Die vorliegende Erfindung wird anhand der beigefügten Figuren näher erläutert, ohne jedoch auf die dort abgebildeten speziellen Ausführungsformen beschränkt zu sein.

Dabei zeigen
- Fig. 1: eine Kondensationsvorrichtung, und
- Fig. 2: eine Ausführungsform einer Polymerisations- vorrichtung anhand eines Fließbildes einer typischen Verfahrensführung zur Herstellung von Polylactid, ausgehend von Milchsäure.

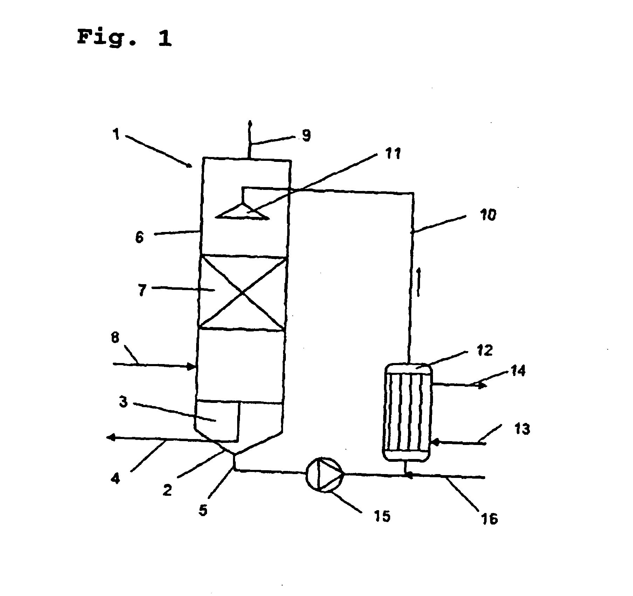

Eine Kondensationsvorrichtung 1, deren Prinzip in Fig. 1 gezeigt ist, enthält einen Kolonnenschuss 6 mit einem Nenndurchmesser von 200 mm. In diesem Schuss ist eine Füllkörperschüttung 7 aus Pall-Ringen mit der Abmessung 15 mm angeordnet. Die Höhe der Schüttung beträgt 500 mm. In den Sumpfbehälter 2 werden 60 1 handelsübliche Milchsäure (Purae HS88) mit einem Wassergehalt von 12 % als Kondensations- und Waschflüssigkeit 3 eingefüllt. Die Milchsäure wird mit einer Pumpe 15 aus dem Sumpf abgezogen, durch einen Wärmeaustauscher 12 über eine Rohrleitung 10 in den Kolonnenschuss 6 zurückgefördert und dort mit einem Flüssigkeitsverteiler 11 gleichmäßig über die Füllkörperschüttung 7 verteilt. Beispielsweise kann der Flüssigkeitsverteiler 11 in Form eines Sprinklers ausgestaltet sein. Der Wärmetauscher 12 temperiert die Flüssigkeit mit einem Kühlmedium 13 bzw. 14, hier Ethylenglykol, auf 30 °C.

Im Kolonnenschuss 6 ist oberhalb der Füllkörperschüttung 7 und der Flüssigkeitsverteilung 11 ein Rohrstutzen 9 angeordnet, der zur Abführung der nicht kondensierbaren Gase und Dämpfe dient. Er ist über eine (in Fig. 1 nicht dargestellte) Kühlfalle, die z. B. mit Trockeneis auf ca. -50 °C gekühlt wird, mit einer Vakuumpumpe verbunden.

Die Vorrichtung 1 wird zum Entwässern unter ein Vakuum bzw. verringerten Druck von 10 mbar gesetzt. Danach wird der Sumpf bis zum auf Höhe des Ablasses 4 befindlichen Überlauf abgelassen. Die Kondensationsvorrichtung 1 ist Teil einer kontinuierlichen Anlage zur Herstellung von Polylactid durch Ringöffnungspolymerisation. Die oben beschriebene Vorgehensweise ist Teil der Anfahrprozedur dieser Anlage. Nachdem auch die übrigen Prozessstufen der Anlage in Betrieb genommen worden sind, wird der Kondensationsvorrichtung 1 kontinuierlich ein Dampfstrom über Stutzen 8 zugeführt, der aus der thermischen Depolymerisation eines Milchsäure-Oligomers mit einer mittleren Molmasse Mₙ von 1.500 g/mol kommt und aus dem die Hauptmenge an Dilactid durch einen Oberflächenkondensator bereits auskondensiert wurde. Der Dampfstrom enthält Stickstoff, Wasser, Milchsäure und restliches Dilactid und hat eine Temperatur von 140 °C. Nach Eintritt in die Kondensationsvorrichtung 1 strömt er entsprechend dem Druckgefälle im Gegenstrom zu der auf 30 °C temperierten Flüssigkeit 3 durch die Füllkörperschüttung 7. Dabei wird ein Großteil der mitgeführten Komponenten entweder kondensiert oder ausgewaschen. Die nicht kondensierbaren Reste verlassen zusammen mit dem enthaltenen Stickstoff durch den Gas-Auslass 9 die Kondensationsvorrichtung 1 und werden in der folgenden Kühlfalle restlos niedergeschlagen, wobei der Stickstoff durch die Vakuumpumpe abgezogen wird.

Zur Ermittlung der Mengenströme des Dampfes und der kondensierten und der nichtkondensierten Anteile lässt man das Flüssigkeitsniveau im Sumpf 2 über 24 Stunden ansteigen. Danach entleert man den Sumpf bis auf den Füllstand vor Beginn der Dampfeinleitung (Überlauf). Die Menge an aufgefangenem Kondensat beträgt 5,9 kg, der Wassergehalt wird durch Karl-Fischer-Titration mit 2 Gew.-% bestimmt. Zur gleichen Zeit wird die Kühlfalle vor der Vakuumpumpe gewechselt und der Inhalt gewogen. Es haben sich 0,9 kg niedergeschlagen, der Wassergehalt wird mit 90 % bestimmt. Dilactid konnte durch HPLC-Analyse nicht festgestellt werden. Die Vakuumpumpe zeigt keinen Leistungsverlust, der auf Verschleiß oder Korrosion deuten würde. Gegebenenfalls kann über die Zuführung 16 frische wässrige Milchsäure in den Kreislauf eingebracht werden.

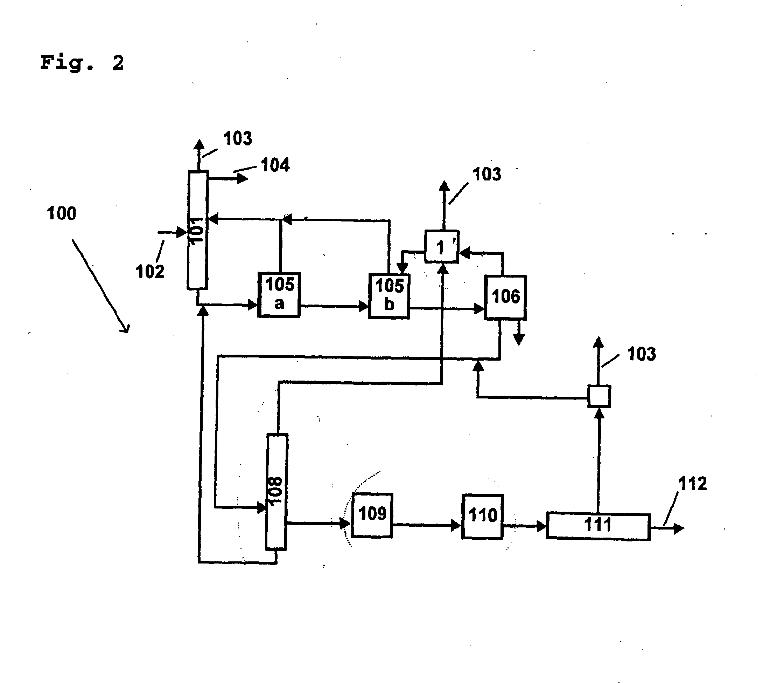

In Fig. 2 ist der kontinuierliche Gesamtprozess der Polylactidherstellung (PLA-Prozess) ausgehend von Milchsäure dargestellt. Der Prozess untergliedert sich dabei in die folgenden Teilschritte, die mit den in die Polymerisationsvorrichtung 100 integrierten, im Folgenden näher erläuterten Einzelbestandteilen ausgeführt werden. Die Polymerisationsvorrichtung 100 umfasst dabei eine Kondensationsvorrichtung 1.

### 1. Aufkonzentration von Milchsäure

Das Ausgangsmaterial für den Prozess ist Milchsäure. Dabei muss der Gehalt an Milchsäure höher als 80 Gew.-% sein. Vorzugsweise beträgt dabei die Milchsäurekonzentration mehr als 90 %, weil das Wasser vor der Polymerisation entfernt werden muss. Die Trennung von Wasser und Milchsäure wird dabei in einer Rektifikationssäule 101 vorgenommen. Dabei wird über einen Absaugstutzen 103 Vakuum angelegt, das dampfförmig anfallende Wasser wird kondensiert und über einen weiteren Stutzen 104 kopfseitig entnommen. Die Zuführung der Milchsäure erfolgt dabei kontinuierlich über einen weiteren Stutzen 102. Das Destillat ist reines Wasser, das sumpfseitig anfallende Produkt ist Milchsäure mit einer Konzentration von mehr als 99 Gew.-%.

Neben der Abtrennung von Wasser aus dem Ursprungsmaterial (Milchsäure) dient die Rektifikationssäule 101 ebenso zur Trennung der Dämpfe aus den Präkondensations-Reaktoren 105a und 105b. Die Dampfströme bestehen dabei aus Milchsäure, Lactoylmilchsäure, Dilactid und Wasser. Das Wasser wird kopfseitig abgezogen, Milchsäure und ihre Derivate gehen in den Sumpf der Rektifikatiönssäule und von dort zusammen mit der aufkonzentrierten Milchsäure in den ersten Präkondensations-Reaktor 105a.

### 2. Präkondensation

Die aufkonzentrierte Milchsäure wird in einer Serie von zwei Reaktoren 105a und 105b durch Polykondensation in ein Präpolymer überführt. Die Polykondensation läuft unter zwei verschiedenen Drücken und Temperaturen ab, um den Reaktionsumsatz zu optimieren. Im ersten Reaktor 105a sind die Konditionen so gewählt, dass die Verdampfung von Milchsäure minimiert ist und gleichzeitig die Entfernung von Wasser erleichtert wird. Im zweiten Schritt der Polykondensation ist die Reaktionsgeschwindigkeit durch eine höhere Temperatur erhöht, gleichzeitig wird der Druck vermindert, um die Wasserkonzentration in der Schmelze weiter zu mindern. Die mittlere Molmasse (Zahlenmittel) des Präpolymers liegt dabei zwischen 500 und 2.000 g/mol.

### 3. Cyclisierende Depolymerisation

Das Präpolymer steht in chemischem Gleichgewicht mit dem cyclischen Dimer der Milchsäure, dem Dilactid. Durch Einstellung von Druck und Temperatur im Depolymerisationsreaktor 106 ist gewährleistet, dass das Lactid kontinuierlich aus dem Präpolymer gebildet wird und verdampft. Der Dampfstrom aus dem Depolymerisationsreaktor 106 besteht hauptsächlich aus Lactid. Wasser, Milchsäure und deren lineare Oligomere sind nur in untergeordneten Mengen vorhanden. Die Dämpfe werden teilweise in der Kondensationsvorrichtung 1 kondensiert: Wasser und der größte Anteil an Milchsäure bleiben dabei dampfförmig. Das Kondensat enthält zuvorderst das Lactid, Lactoylmilchsäure (das lineare Dimer der Milchsäure) und höhere lineare Oligomere. [Lactid liegt in zwei stereoisomeren Formen vor: das optisch aktive *L,L*-Lactid und das Mesolactid, aus einer Kombination einer L(+)- und *D*(-)-Milchsäureeinheit. Die *D*(-)-Einheiten stammen teils aus dem Edukt, teils werden sie durch Racemisierung von *L*(+)-Einheiten während der Präpolymerisation und der Depolymerisation gebildet.]

### 4. Lactid-Reinigung

Während der Ringöffnungspolymerisation hängt das erreichbare Molekulargewicht und somit bedeutende mechanische Eigenschaften des Polylactids vom Reinheitsgrad des Lactids ab. Die Hydroxyl-Gruppen der als Verunreinigung enthaltenen Milchsäure und Lactoylmilchsäure dienen dabei als Ausgangspunkt der Polymerisation. Je höher die Konzentration der Hydroxyl-Gruppen im Lactid ist, desto geringer fällt das erreichbare Molekulargewicht des Polymers aus. Die Konzentration der Hydroxyl-Gruppen im Rohlactid ist nach der cyclisierenden Depolymerisation zu hoch. Das kondensierte Lactid wird in einer Rektifikationssäule oder einer Trennwandkolonne 108 bis zur benötigten Hydroxylgruppenkonzentration aufgereinigt. Das gereinigte Lactid wird der Säule 108 als Seitenprodukt entnommen. Das Destillat und das Sumpfprodukt werden dem Prozess an unterschiedlichen Stellen wieder zugeführt. Neben dem Molekulargewicht des Polylactids werden seine Eigenschaften stark durch den D-Gehalt (die Menge an strukturellen Einheiten, die die D-Konfiguration aufweisen) beeinflusst.

### 5. Ringöffnungspolymerisation

Die Ringöffnungspolymerisation wird in einem Reaktor unternommen, der aus einer Kombination eines Rührkessels 109 und eines Rohrreaktors 110 gebildet ist. Im ersten Reaktor 109 wird das niedrigviskose Lactid zu PLA polymerisiert mit einer Umsetzungsrate von ca. 50 %. Katalysator und Additive werden homogen in die Schmelze eingemischt.

Im Rohreaktor 110 wird die Polymerisationsreaktion so lange fortgeführt, bis ein chemisches Gleichgewicht zwischen Polymer und Monomer erreicht wird. Die maximale Umsetzung des Monomers beträgt ca. 95 %. Während der Polymerisation erhöht sich die Viskosität auf ca. 10.000 pa·sec.

### 6. Entmonomerisierung

Um ein stabiles Polylactid zu erhalten, ist die Monomerkonzentration von ungefähr 5 Gew.-% in der Schmelze zu hoch. Deswegen muss eine Entmonomerisierung durchgeführt werden. Dies wird durch eine Entgasung der Schmelze in einem Doppelschneckenextruder 111 erreicht. Aufgrund der Tatsache, dass die Ringöffnungspolymerisation eine Gleichgewichtsreaktion ist, wird ein Stabilisator vor der Entmonomerisierung zugegeben, um die Rückbildung des Monomers während und nach der Entgasung zu verhindern.

### 7. Granulierung und Kristallisation

Anschließend an die Entmonomerisierung wird die Schmelze dem Extruder 111 entnommen und in ein Granulat 112 überführt. Dabei können sowohl Stranggranulation oder Unterwasser-Granulation durchgeführt werden. In beiden Fällen muss das PLA-Granulat vor der Trocknung und der Verpackung kristallisiert werden. Die Kristallisation wird bei erhöhten Temperaturen und unter Rühren durchgeführt, solange bis das Granulat nicht mehr aneinander klebt.

Eine im Voranstehenden beschriebene Kondensationsvorrichtung 1 kann beispielsweise dazu verwendet werden, um in einem in Fig. 2 dargestellten Prozess Dilactid-Dämpfe aus den einzelnen Prozessstufen abzutrennen. Dazu wird die Kondensationsvorrichtung bevorzugt als integraler Bestandteil einer in Fig. 2 dargestellten Anordnung verwendet. Eine Zuführung von Prozessdämpfen zur Kondensationsvorrichtung 1 kann aus einer, mehreren oder allen Prozessstufen erfolgen. Somit ist die Anordnung der Kondensationsvorrichtung nicht auf die in Fig. 2 abgebildete Anordnung beschränkt, die Kondensationsvorrichtung 1 kann ebenso anderen Prozessstufen nach und/oder vorgeschaltet sein.

## Patentansprüche

1. Verfahren zur Kondensation und/oder Wäsche eines dampfförmigen bioabbaubaren, intermolekularen cyclischen Diesters einer alpha-Hydroxycarbonsäure der Formel II, 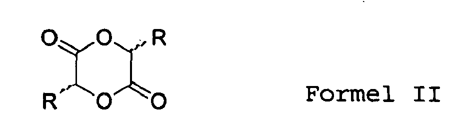 wobei R ausgewählt ist aus Wasserstoff oder linearen oder verzweigten aliphatischen Resten mit 1 bis 6 Kohlenstoffatomen,
aus einem Dampfgemisch, enthaltend den Diester der Formel II, die zum Diester der Formel II korrespondierende alpha-Hydroxycarbonsäure der Formel I und Wasser durch zumindest einmaliges Inkontaktbringen eines Stromes einer Kondensations- und Waschflüssigkeit (3), enthaltend eine wässrige Lösung der zum Diester der Formel II korrespondierenden alpha-Hydroxycarbonsäure der Formel I 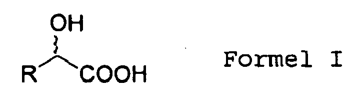 mit dem Dampfgemisch, wobei der im Dampfgemisch enthaltene Diester der Formel II in der Kondensations- und Waschflüssigkeit (3) gelöst wird.

2. Verfahren nach Anspruch 1, **dadurch gekennzeichnet, dass** die Kondensations- und Waschflüssigkeit (3) in einem Kreislauf geführt wird.

3. Verfahren nach Anspruch 1 oder 2, **dadurch gekennzeichnet, dass** die Kondensations- und Waschflüssigkeit (3) vor Inkontaktbringen auf eine Temperatur zwischen 10 °C und 80 °C, bevorzugt zwischen 15 °C und 60 °C eingestellt wird.

4. Verfahren nach einem der Ansprüche 1 bis 3, **dadurch gekennzeichnet, dass** der Kondensations-und Waschflüssigkeit (3) soviel eines Gemisches aus Wasser und Hydroxycarbonsäure der Formel I zugesetzt wird, dass die Löslichkeitsgrenze des Diesters der Formel II in der Kondensations- und Waschflüssigkeit nicht überschritten wird.

5. Verfahren nach einem der Ansprüche 1 bis 4, **dadurch gekennzeichnet, dass** der Kondensations-und Waschflüssigkeit (3) soviel eines Gemisches aus Wasser und Hydroxycarbonsäure der Formel I zugesetzt wird, dass die Viskosität der Kondensations- und Waschflüssigkeit annähernd konstant bleibt.

6. Verfahren nach einem der Ansprüche 1 bis 5, **dadurch gekennzeichnet, dass** die Konzentration des Diesters der Formel II in der Kondensations- und Waschflüssigkeit (3) auf maximal 5 Gew.-% eingestellt wird.

7. Verfahren nach einem der Ansprüche 1 bis 6, **dadurch gekennzeichnet, dass** portionsweise oder kontinuierlich frische wässrige Lösung der alpha-Hydroxycarbonsäure der Formel I zur Kondensations- und Waschflüssigkeit (3) zugegeben wird.

8. Verfahren nach einem der Ansprüche 1 bis 7, **dadurch gekennzeichnet, dass** nach Erreichen einer Konzentration von höchstens 5 Gew.-%, bevorzugt höchstens 3 Gew.-% des Diesters der Formel II in der Kondensations- und Waschflüssigkeit (3) eine zumindest teilweise Entnahme der Kondensations-und Waschflüssigkeit (3) erfolgt.

9. Verfahren nach vorhergehendem Anspruch, **dadurch gekennzeichnet, dass** die Entnahme portionsweise oder kontinuierlich erfolgt.

10. Verfahren nach einem der Ansprüche 1 bis 9, **dadurch gekennzeichnet, dass** das Inkontaktbringen bei einem Druck zwischen 5 mbar und 900 mbar, bevorzugt zwischen 10 mbar und 200 mbar erfolgt.

11. Verfahren nach einem der Ansprüche 1 bis 10, **dadurch gekennzeichnet, dass** der Diester der Formel II 3,6-Dimethyl-1,4-dioxan-2,5-dion (Dilactid) und die alpha-Hydroxycarbonsäure der Formel I Milchsäure ist.

12. Verfahren nach vorhergehendem Anspruch, **dadurch gekennzeichnet, dass** das Dilactid L,L-Dilactid und die Milchsäure L-Milchsäure ist.

13. Verfahren nach einem der Ansprüche 1 bis 12, **dadurch gekennzeichnet, dass** zumindest ein Teil des Diesters der Formel II aus einer vorgeschalteten Reinigungsvorrichtung stammt.

14. Verfahren nach einem der Ansprüche 1 bis 13, **dadurch gekennzeichnet, dass** zumindest ein Teil des Dampfgemisches mindestens aus einer Prozess-stufe der Herstellung von Polylactid, der Polykondensation von Milchsäure, der thermischen Depolymerisation von Oligomeren der Milchsäure mit einer mittleren Molmasse zwischen 500 g/mol und 5000 g/mol, der Rektifikation von Dilactid, der ringöffnenden Polymerisation eines dilactidhaltigen Reaktionsgemisches, der Vakuum-Entmonomerisierung von Polylactid oder dessen Copolymeren, aus zwei oder mehr Prozessstufen der zuvor genannten Verfahren und/oder aus mehreren der zuvor genannten Verfahren gleichzeitig stammt.

15. Verfahren nach einem der Ansprüche 1 bis 14 zur Herstellung von bioabbaubaren, intermolekularen cyclischen Diestern einer alpha-Hydroxycarbonsäure der Formel II, bevorzugt Dilactid, besonders bevorzugt *L*-Dilactid.

16. Verfahren nach einem der Ansprüche 1 bis 14 zur Herstellung von Polymeren von cyclischen Diestern einer alpha-Hydroxycarbonsäure der Formel II, bevorzugt Polylactid (PLA), besonders bevorzugt *L*-Polylactid (PLLA).

## Claims

1. Process for the condensation and/or washing of a vaporous biodegradable,
intermolecular cyclic diester of an alpha-hydroxycarboxylic acid of the formula II 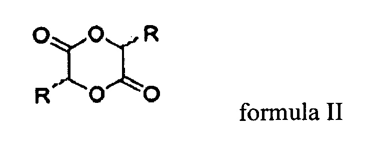 wherein R is chosen from hydrogen or linear or branched aliphatic radicals having 1 to 6 carbon atoms,
from a vapour mixture containing the diester of the formula II, the alpha-hydroxycarboxylic acid of the formula I corresponding to the diester of the formula II and water, by bringing into contact at least once a stream of a condensation and washing liquid (3) containing an aqueous solution of the alpha-hydroxycarboxylic acid of the formula I corresponding to the diester of the formula II 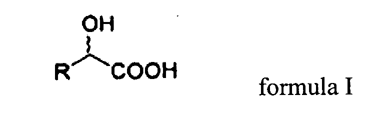 with the vapour mixture, the diester of the formula II contained in the vapour mixture being dissolved in the condensation and washing liquid (3).

2. Process according to claim 1, **characterized in that** the condensation and washing liquid (3) is led in a circulation.

3. Process according to claim 1 or 2, **characterized in that** the condensation and washing liquid (3) is adjusted to a temperature of between 10 °C and 80 °C, preferably between 15 °C and 60 °C, before the bringing into contact.

4. Process according to one of claims 1 to 3, **characterized in that** a mixture of water and hydroxycarboxylic acid of the formula I is added to the condensation and washing liquid (3) in an amount such that the solubility limit of the diester of the formula II in the condensation and washing liquid is not exceeded.

5. Process according to one of claims 1 to 4, **characterized in that** a mixture of water and hydroxycarboxylic acid of the formula I is added to the condensation and washing liquid (3) in an amount such that the viscosity of the condensation and washing liquid remains approximately constant.

6. Process according to one of claims 1 to 5, **characterized in that** the concentration of the diester of the formula II in the condensation and washing liquid (3) is adjusted to a maximum of 5 wt.%.

7. Process according to one of claims 1 to 6, **characterized in that** fresh aqueous solution of the alpha-hydroxycarboxylic acid of the formula I is added in portions or continuously to the condensation and washing liquid (3).

8. Process according to one of claims 1 to 7, **characterized in that** when a concentration of at most 5 wt.%, preferably at most 3 wt.% of the diester of the formula II in the condensation and washing liquid (3) is reached, an at least partial removal of the condensation and washing liquid (3) is carried out.

9. Process according to the preceding claim, **characterized in that** the removal is carried out in portions or continuously.

10. Process according to one of claims 1 to 9, **characterized in that** the bringing into contact is carried out under a pressure of between 5 mbar and 900 mbar, preferably between 10 mbar and 200 mbar.

11. Process according to one of claims 1 to 10, **characterized in that** the diester of the formula II is 3,6-dimethyl-1,4-dioxane-2,5-dione (dilactide) and the alpha-hydroxycarboxylic acid of the formula I is lactic acid.

12. Process according to the preceding claim, **characterized in that that** the dilactide is *L,L*-dilactide and the lactic acid is *L*-lactic acid.

13. Process according to one of claims 1 to 12, **characterized in that** at least a part of the diester of the formula II originates from an upstream purification device.

14. Process according to one of claims 1 to 13, **characterized in that** at least a part of the vapour mixture originates at least from a process stage of the preparation of polylactide, the polycondensation of lactic acid, the thermal depolymerisation of oligomers of lactic acid with an average molecular weight of between 500 g/mol and 5,000 g/mol, the rectification of dilactide, the ring-opening polymerization of a dilactide-containing reaction mixture, the vacuum demonomerization of polylactide or copolymers thereof, from two or more process stages of the abovementioned processes and/or from several of the abovementioned processes simultaneously.

15. Process according to one of claims 1 to 14 for the preparation of biodegradable, intermolecular cyclic diesters of an alpha-hydroxycarboxylic acid of the formula II, preferably dilactide, particularly preferably *L*-dilactide.

16. Process according to one of claims 1 to 14 for the preparation of polymers of cyclic diesters of an alpha-hydroxycarboxylic acid of the formula II, preferably polylactide (PLA), particularly preferably *L*-polylactide (PLLA).

## Revendications

1. Procédé pour la condensation et/ou le lavage d'un diester cyclique intermoléculaire biodégradable, sous forme de vapeur, d'un acide alpha-hydroxycarboxylique de formule II suivante : 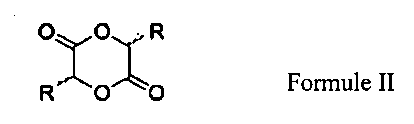 dans laquelle R est choisi parmi l'hydrogène ou des radicaux aliphatiques linéaires ou ramifiés ayant 1 à 6 atomes de carbone,
à partir d'un mélange en phase vapeur, contenant le diester de formule II, l'acide alpha-hydroxycarboxylique de formule I correspondant au diester de formule II et de l'eau, par au moins une mise en contact d'un courant de liquide de condensation et de lavage (3), contenant une solution aqueuse de l'acide alpha-hydroxycarboxylique de formule I suivante, correspondant au diester de formule II : 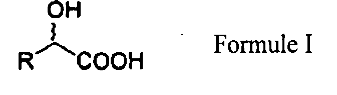 avec le mélange en phase vapeur, dans lequel le diester de formule II contenu dans le mélange en phase vapeur est dissous dans le liquide de condensation et de lavage (3).

2. Procédé selon la revendication 1, **caractérisé en ce que** le liquide de condensation et de lavage (3) est conduit dans un circuit.

3. Procédé selon la revendication 1 ou 2, **caractérisé en ce que** le liquide de condensation et de lavage (3) est ajusté, avant la mise en contact, à une température comprise entre 10 °C et 80 °C, de préférence, entre 15 °C et 60 °C.

4. Procédé selon l'une quelconque des revendications 1 à 3, **caractérisé en ce que** le liquide de condensation et de lavage (3) est ajouté à un mélange constitué d'eau et d'acide hydroxycarboxylique de formule I en quantité suffisante pour ne pas dépasser la limite de solubilité du diester de formule II dans le liquide de condensation et de lavage.

5. Procédé selon l'une quelconque des revendications 1 à 4, **caractérisé en ce que** le liquide de condensation et de lavage (3) est ajouté à un mélange constitué d'eau et d'acide hydroxycarboxylique de formule I en quantité suffisante pour que la viscosité du liquide de condensation et de lavage demeure presque constante.

6. Procédé selon l'une quelconque des revendications 1 à 5, **caractérisé en ce que** la concentration du diester de formule II dans le liquide de condensation et de lavage (3) est ajustée à 5 % en poids au maximum.

7. Procédé selon l'une quelconque des revendications 1 à 6, **caractérisé en ce que** l'on ajoute une solution aqueuse fraîche de l'acide alpha-hydroxycarboxylique de formule I par fraction ou en continu au liquide de condensation et de lavage (3).

8. Procédé selon l'une quelconque des revendications 1 à 7, **caractérisé en ce que**, après avoir atteint une concentration de 5 % en poids au maximum, de préférence de 3 % en poids au maximum du diester de formule II dans le liquide de condensation et de lavage (3), on effectue un soutirage au moins partiel du liquide de condensation et de lavage (3).

9. Procédé selon la revendication précédente, **caractérisé en ce que** le soutirage est effectué par fraction ou en continu.

10. Procédé selon l'une quelconque des revendications 1 à 9, **caractérisé en ce que** la mise en contact est faite à une pression comprise entre 5 mbars et 900 mbars, de préférence, entre 10 mbars et 200 mbars.

11. Procédé selon l'une quelconque des revendications 1 à 10, **caractérisé en ce que** le diester de formule II est la 3,6-diméthyl-1,4-dioxan-2,5-dione (dilactide) et l'acide alpha-hydroxycarboxylique de formule I est l'acide lactique.

12. Procédé selon la revendication précédente, **caractérisé en ce que** le dilactide est un L,L-dilactide et l'acide lactique et un acide lactique de configuration L.

13. Procédé selon l'une quelconque des revendications 1 à 12, **caractérisé en ce qu'**au moins une partie du diester de formule II provient d'un dispositif de purification installé en amont.

14. Procédé selon l'une quelconque des revendications 1 à 13, **caractérisé en ce qu'**au moins une partie du mélange en phase vapeur provient en même temps d'au moins une étape de procédé de la fabrication de polylactide, de la polycondensation de l'acide lactique, de la dépolymérisation thermique d'oligomères de l'acide lactique ayant une masse molaire moyenne comprise entre 500 g/mole et 5000 g/mole, de la rectification du dilactide, de la polymérisation par ouverture de cycle d'un mélange réactionnel contenant du dilactide, de l'élimination sous vide de monomères de polylactide ou de ses copolymères, de deux étapes de procédé ou plus du procédé précité et/ou de plusieurs des procédés précités.

15. Procédé selon l'une quelconque des revendications 1 à 14, pour fabriquer des diesters cycliques intermoléculaires biodégradables d'un acide alpha-hydroxycarboxylique de formule II, de préférence, un dilactide, mieux encore, un L-dilactide.

16. Procédé selon l'une quelconque des revendications 1 à 14, pour fabriquer des polymères de diesters cycliques d'un acide alpha-hydroxycarboxylique de formule II, de préférence, un polylactide (PLA), mieux encore, un L-polylactide (PLLA).
